# EUROPEAN PATENT SPECIFICATION

(11) **EP 1 802 355 B1**
(45) Date of publication and mention of the grant of the patent: **08.06.2011**
(21) Application number: 04791733.1
(22) Date of filing: 21.10.2004
(51) Int. Cl.: A61L 9/12, A61L 9/20

(54) **AIR CONDITIONING SYSTEM**
KLIMAANLAGE
AIR CONDITIONNE

(43) Date of publication of application: 04.07.2007
(73) Proprietor: CARRIER CORPORATION, Farmington, Connecticut 06034-4015 (US)
(72) Inventor: JOSSERAND, Olivier Pierre, 01122 Montluel (FR); HENRIO, Olivier, 01122 Montluel (FR)
(74) Representative: Booth, Catherine Louise
(86) International application number: PCT/IB2004/003458
(87) International publication number: WO 2006/043124

(56) References cited:
- WO-A-96/37281
- WO-A-03/068273
- GB-A- 2 022 979
- US-A- 5 656 242
- US-A- 5 835 840
- US-B1- 6 468 433
- PATENT ABSTRACTS OF JAPAN vol. 2000, no. 01, 31 January 2000 (2000-01-31) & JP 11 276569 A (SANYO ELECTRIC CO LTD), 12 October 1999 (1999-10-12)
- PATENT ABSTRACTS OF JAPAN vol. 2000, no. 21, 3 August 2001 (2001-08-03) & JP 2001 087361 A (MAX CO LTD), 3 April 2001 (2001-04-03)

## Description

The present invention relates to a fan unit and in particular to a fan unit in which the air is purified using an air purifying lamp.

Fan units for moving and circulating air, particularly in air conditioning systems, typically comprise a rotatable fan body arranged in a housing for moving the air through the housing to a duct for distribution. The air may contain a variety of impurities such as odours, volatile organic compounds (VOCs) and bioaerosols, which typically include bacteria, moulds, spores and viruses etc.. Therefore it may be desirable to purify the air before it is distributed by the duct.

In prior art air conditioning systems, purification of air is performed in a separate stage of the system, prior to or after passage of air through the fan unit. Typically purification is achieved by flowing the air through filters, such as media filters, for removing dust and particulate matter. In addition, the air may be purified biocidally or photocatalytically. In a biocidal system, air is irradiated with, for example, UV radiation having an appropriate wavelength to destroy biological impurities. A photocatalytic purifying system is described in EP-A-1281431. Here, air is purified using a photocatalytic purifier module in an air conditioning unit upstream of the fan. The purifier module comprises a photocatalyst disposed on a filter and an ultraviolet (UV) light source to activate the photocatalyst and oxidise the impurities. This system can provide more efficient purification than previous media filter systems. However, in order to improve the efficiency of the purification, two or more photocatalytic filters and corresponding UV lamps may be required, which can occupy a large volume and thus increase the overall size of the unit.

More widespread use of air conditioning units, extractor fans and other fan units necessitates smaller and more compact units and therefore it is desirable that the modules in a unit are as small as possible and that the number of modules is kept to a minimum.

JP-A-11276569 discloses an air conditioning system in accordance with the preamble of claim 1.

It is an object of the present invention to provide a compact apparatus for purifying air that is particularly, but not exclusively, suited for use in an air conditioning system.

In accordance with a first aspect of the present invention, there is provided an air conditioning system as claimed in claim 1.

Therefore there is provided a fan unit that is capable of destroying bioaerosol impurities in the air, such as bacteria and mould, but that does not require the fan housing or the fan body to be significantly increased in size. Furthermore, by disposing the lamp at least partially within the flow inlet cavity of the fan body, exposure of other components of the fan unit (or of the system in which the fan unit is installed) to the radiation emitted from the lamp is significantly reduced. This is particularly advantageous for any plastic components which may suffer damage from the radiation.

In a preferred embodiment of the present invention, the lamp comprises an ultraviolet (UV) light source. The UV light is preferably emitted with a wavelength in the range of about 40 nm to about 400 nm. More preferably the UV light source emits UV-C light, such as light with a wavelength within the range of about 100 nm to about 280 nm, more preferably of about 250 nm to about 260 nm. Light having a wavelength in these ranges is particularly effective at deactivating or killing bacteria and mould. In a particularly preferred embodiment, the light has a wavelength of about 253.7 nm.

The flow inlet cavity of the fan body is preferably aligned substantially on, or coaxial with, the axis of rotation of the fan body. The cavity may, therefore, be a cylindrical or annular cavity aligned generally coaxially with the axis of rotation of the fan body. The inlet cavity may be any suitable shape and size and the cavity dimensions may depend, for example, on the type of fan body in the fan unit, and/or on the size and shape of the lamp.

As discussed above, the air purifying lamp may be any lamp suitable for purifying the air in fan unit. Many UV lamps are known in the art and would be suitable for use in the fan unit of the present invention. In a preferred embodiment, the lamp may be elongate and aligned generally along or parallel to the axis of rotation of the fan body. Such a lamp would be particularly suitable for use in a centrifugal fan having a cylindrical cavity, such as a blower fan. In an alternative preferred embodiment, the lamp may be generally annular and preferably aligned generally coaxially with the axis of rotation of the fan body. Such a lamp would be particularly suitable for use in a radial fan having an annular cavity in the fan body.

The fan unit air purifier in accordance with the present invention can be used in a wide variety of applications in which it is desired to purify air flowing through a fan. In a particularly preferred embodiment, the fan unit is disposed in a heating, ventilating and air conditioning (HVAC) unit or within a refrigeration unit such as a fridge, a container, a truck, a trailer, a display case etc..

The fan body comprises a blower wheel. The blower wheel comprises a pleated filter. In this embodiment, the filter can remove particulate impurities to complement the biocidal effects of the lamp, thereby providing improved purity distributed air.

The housing can be any suitable shape, size and configuration and its dimensions may depend on the intended purpose of the fan unit. In a preferred embodiment, particularly where the fan unit is a blower fan in an HVAC unit for example, the housing is a scroll-shaped housing.

Whilst radiation from the air purifying lamp, particularly UV light, can be effective at destroying or deactivating certain bacteria and moulds, under certain conditions the purification of the air by light only may not destroy all the impurities that are required to be removed from the air. Therefore, in a preferred embodiment, the fan unit further comprises a photocatalytic coating disposed on at least a part of the fan unit. The photocatalytic coating is activated by the light from the lamp and oxidises impurities such as odours,VOCs and bioaerosols in the air.

This is in itself a novel concept in broad terms, so from a further aspect, the invention provides an air purifier comprising: a fan unit comprising a fan housing and a rotatable fan body disposed within the fan housing; a photocatalytic medium disposed on the fan housing or rotatable fan body; and a lamp arranged to illuminate the said medium.

Preferably the lamp comprises an ultraviolet (UV) light source. The UV light is preferably emitted with a wavelength in the range of about 40 nm to about 400 nm. More preferably the UV light source emits UV-C light, such as light with a wavelength within the range of about 100 nm to about 280 nm, more preferably of about 250 nm to about 260 nm. Light having a wavelength in these ranges is particularly effective at deactivating or killing bacteria and mould and also at activating the photocatalytic coating. In a particularly preferred embodiment, the light has a wavelength of about 253.7 nm. In an alternative preferred embodiment, the lamp comprises a UV-A and/or a UV-B light source. Preferably the lamp emits light having a wavelength within the range of about 260 nm to about 400 nm, more preferably of about 320 nm to about 400 nm or of about 260 nm to about 300 nm or any suitable wavelength within these ranges. UV-A/B light is particularly effective at activating the photocatalytic medium and thereby degrading impurities in the air within the fan housing.

In the low static pressure fan discussed above, a photocatalytic coating may be disposed on the pleated filter at least.

As discussed above, the efficiency of the fan unit in purifying the air may be related to the surface area of the photocatalytic coating that is exposed to the photocatalytic lamp and to the air flowing through the fan unit. Since the air is purified at least partially by the reaction between the activated photocatalyst and the impurities in the air, then increasing the surface area of the coating exposed to the lamp and to the air should increase the purity of the air. Therefore in a preferred embodiment of the present invention, the inner surface of the housing may have a photocatalytic coating disposed thereon.

The area of the photocatalytic coating present in the fan unit can be further increased by providing the housing with a profiled surface to increase the surface area of the housing. The housing may, therefore be provided with a grooved inner surface, on which a photocatalytic coating is disposed.

There are many photocatalytic coatings known in the art. In a preferred embodiment, the photocatalytic coating comprises titanium dioxide, TiO₂. Titanium dioxide is particularly effective at oxidising impurities when activated with a UV light source, particularly a UV-C light source.

The lamp of the apparatus of the may not be readily visible to a user who may not therefore be able easily to ascertain whether it is functioning properly. Accordingly, in a preferred embodiment means may be provided for remotely monitoring the operational status of the lamp. Such means may comprise an optical fibre. The fibre may be suitably coupled to a display means such as a light emitting diode (LED). The LED may, for example, be placed adjacent a switch the unit so that the user can easily see whether the lamp is working when they switch the unit on. For convenience, the optical fibre could be coupled to the wiring from the switch to the unit. Alternatively, the remote monitoring means may comprise a light dependent resistor (LDR). An LDR is an input transducer. Changes in the brightness of the light shining onto the surface of the LDR result in changes in its resistance. Usually an LDR provides a relatively low resistance (for example a few kΩ) when the light is on and a relatively high resistance (for example several thousand kΩ) when the light is off. If this LDR is connected to the input of a controller, the controller might generate a warning on a thermostat (for example by lighting an LED on the thermostat) or to a Building Management System through a communication bus (for example by sending a message to the system to replace the failed lamp).

The above-mentioned and other features of the various embodiments of the present invention will now be described with reference to the accompanying drawings, in which:
Figure 1 shows a cross-sectional side view of an embodiment of the fan unit in accordance with the present invention;
Figure 2 shows a cross-sectional side view of an alternative embodiment of the fan unit not in accordance with the present invention;
Figure 3 shows a back view of the fan unit of Figure 1 or Figure 2; and
Figure 4 shows a control panel for use with the fan unit of Figure 1 or Figure 2 and Figure 3.

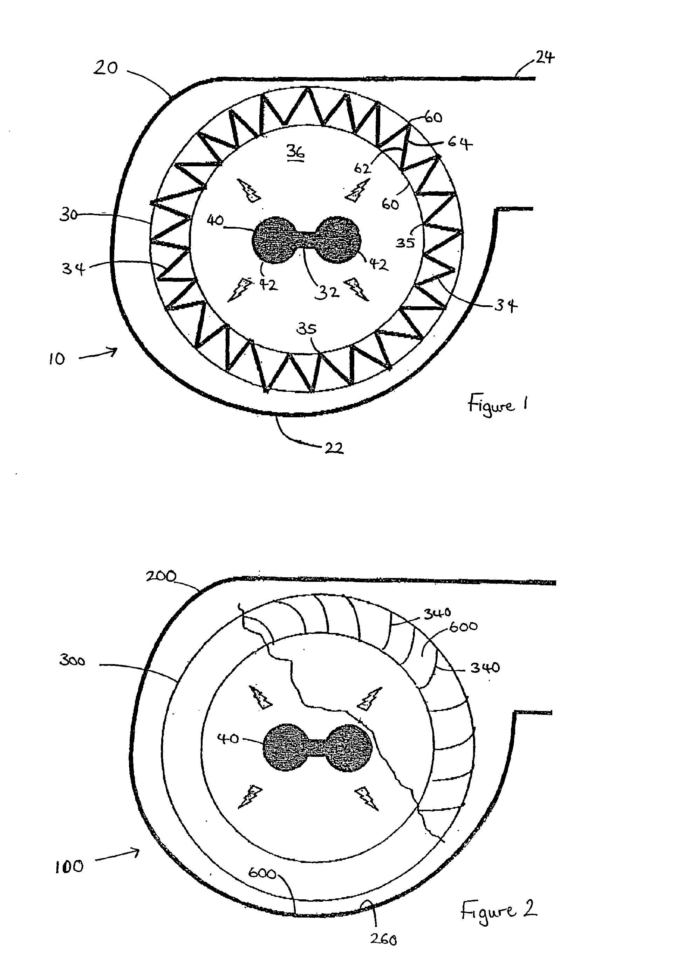

With reference to Figure 1, a blower fan unit 10 in accordance with an embodiment of the present invention is shown.

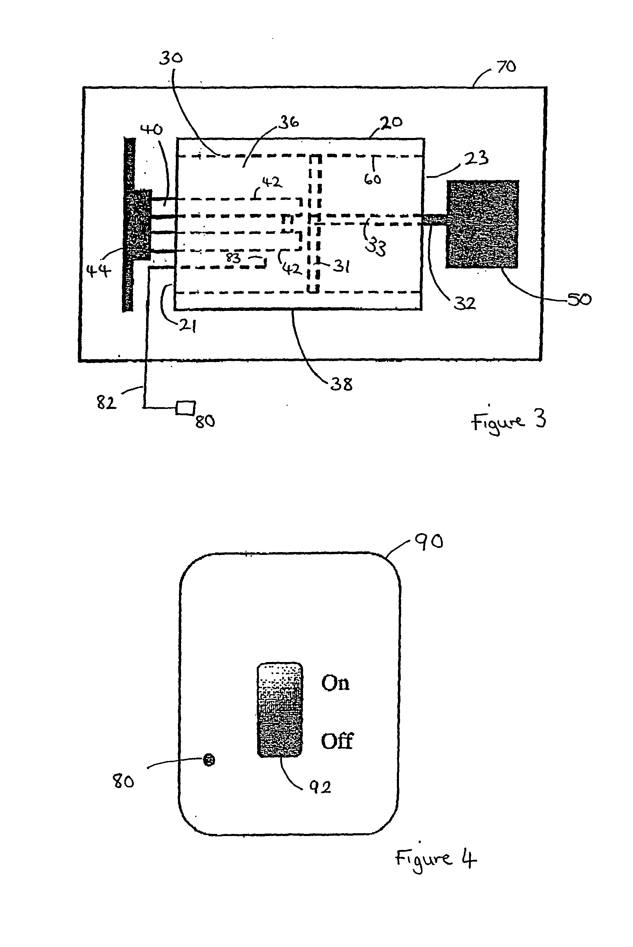

The fan unit comprises a housing 20 which is generally scroll shaped and has a main body 22 and an air exit duct 24. Air is drawn into the housing 20 through opening 21 (shown in Figure 3) on the side of the housing 20 and is blown out of the housing 20 through exit duct 24 as is known in the art. Within the housing is a fan body 30, mounted to rotate about a central axis of rotation 32. For clarity, the mounting is not shown in Figure 1.

In the blower of Figure 1, the fan body comprises a filter 30 having a plurality of pleats 34 and, in use, the fan body 30 rotates about the fan axis 32 and draws air into the flow inlet cavity 36 of the fan body. The flow inlet cavity is defined inwardly of the inner tips 35 of the pleats and is generally cylindrical in shape.

The fan unit further comprises a UV lamp 40 which consists of two, spaced apart, elongate bulb sections 42 as are well known in the art. The lamp 40 is mounted such that it protrudes into the inlet cavity 36. As best seen in Figure 3, most of the length of the lamp 40 is located within the inlet cavity 36, with only a small proportion of the lamp 40 and its mounting and power unit 44 placed outside the cavity 36. The lamp 40 is generally aligned parallel to the axis of rotation 32 of the fan body 30. The lamp 40 is also aligned such that the elongate bulb sections 42 are aligned one either side of the axis of rotation 32 of the fan body 30 and are approximately equidistant from the axis of rotation 32.

The fan body 30 further comprises a photocatalytic coating 60 disposed on the pleated filter. The coating 60 in this embodiment is deposited on the inner and outer surfaces 62, 64 of the pleated filter.

In use, the fan body 30 rotates and the lamp 40 is switched on and irradiates the photocatalytic coating 60 thus activating the catalyst. Air drawn into the housing 20 is drawn into the centre of the fan body 30, via the flow inlet cavity 36 and is blown out of the fan body 30 and out of the housing 20 via exit duct 24. Impurities in the air are destroyed firstly by the biocidal effect of the UV light incident upon them and secondly by oxidation of the impurities as a result of activation of the photocatalyst. Air exiting the duct 24 is therefore sub stantially purified and relatively free of impurities such as bioaerosols, odours, moulds and the like.

With reference to Figure 2, a blower fan unit 100 is shown. The same reference numerals are used in Figure 2 for components having the same construction as those in the embodiment shown in Figure 1.

The arrangement of Figure 2 varies from that of Figure 1 in that the fan body 300 comprises a plurality of radially extending vanes 340 arranged in a conventional manner as is known in the art.

The arrangement shown in Figure 2 also differs from the embodiment of Figure 1 in that the housing 200 comprises a plurality of grooves 260 in the inner surface of the housing 200. Furthermore, the inner surface of the housing 200 is also coated with the photocatalytic coating 600. The additional photocatalytic layer provides a larger reaction surface for the UV light and thus a larger purifying surface for the air.

Figure 3 will now be described with reference to the embodiment on the present invention shown in Figure 1. Figure 3 shows a rear view of the blower of Figures 1 or 2. Features hidden inside the unit are shown in dotted lines.

The fan body 30 is generally cylindrical, having an axis of rotation 32 aligned with the axis of the cylinder. The fan body 30 is mounted to a shaft 33 by conventional means 31. The shaft 33 is powered by a motor 50. The motor is mounted to a support bracket 70 which supports the housing 20 and other components.

The lamp 40 of the fan unit is also mounted to the support bracket 70 by mounting 44. The lamp 40 protrudes into flow inlet cavity 36 of fan body 30 as discussed above.

A fibre optic cable 82 has a first end 83 arranged inside the cavity 36 of the fan 30 and a second end coupled to a light emitting diode 80. The LED 80 is placed away from the housing 20, in a place that is convenient for monitoring the status of the lamp. As shown in Figure 4, the LED 80 can be placed adjacent the switch 92 for the unit. In this way, the operator of the fan unit can readily verify whether the lamp is operating as required.

Although described above in the context of an HVAC system, the principles of the present invention can be incorporated into any air moving system. Applications could include systems for aircraft cabins, train carriages, for placing in false ceilings and in smokers' rooms. The compact construction afforded by the invention is particularly advantageous in these applications.

The fibre optic cable may alternatively or additionally coupled to a sensor which may be located in a controller for the system in which the fan unit is placed, such as an air conditioner controller. This controller may forward the information provided to the controller to a Building Management System. In an alternative embodiment, a light dependent resistor (LDR) may be used instead of the fibre optic cable to detect whether the lamp is operating as required.

## Claims

1. An air conditioning system having a fan unit (10, 100) for purifying air, the fan unit comprising:
a fan housing (20, 200);
a rotatable fan body (30, 300) disposed within the housing, the fan body having a flow inlet cavity (36); and
an air purifying lamp (40) disposed at least partially within the flow inlet cavity, whereby, in use, air drawn into the housing through the flow inlet cavity is purified by radiation emitted by the lamp;
**characterised in that**:
the fan body (30, 300) is a blower wheel which comprises a pleated filter; and
a photocatalytic material (60, 600) is disposed on the fan body (30, 300).

2. The air conditioning system of claim 1, wherein the inner surface of the fan housing (20, 200) is grooved.

3. The air conditioning system of claim 1 or 2 wherein photocatalytic material (60, 600) is also disposed on at least an inner surface of the fan housing (20, 200).

4. The air conditioning system of any preceding claim , wherein the lamp (40) comprises an ultraviolet lamp.

5. The fan air conditioning system of claim 4, wherein the ultraviolet lamp (40) emits light having a wavelength within the range of 100 nm to 280 nm.

6. The air conditioning system of claim 4, wherein the ultraviolet lamp (40) emits light having a wavelength of 253.7 nm.

7. The air conditioning system of claim 1, wherein the lamp (40) emits ultraviolet light having a wavelength within the range of 260 nm to 400 nm.

8. The air conditioning system of claim 1, wherein the lamp (40) emits ultraviolet light having a wavelength within the range of 200 nm to 260 nm, preferably 253.7 nm.

9. The air conditioning system of any preceding claim, wherein the lamp (40) is elongate.

10. The air conditioning system of claim 9 wherein the lamp (40) is aligned generally along or parallel to the axis of rotation of the fan body (30, 300).

11. The air conditioning system of any of claims 1 to 8, wherein the air purifying lamp (40) is annular.

12. The air conditioning system of any preceding claim wherein the fan body (30, 300) is a radial fan wheel.

13. The air conditioning system of any preceding claim, wherein the photocatalytic material (60, 600) comprises titanium dioxide.

14. The air conditioning system of any preceding claim, further comprising means for remotely monitoring the lamp (40) to determine its operational status.

15. The air conditioning system of claim 14, wherein said monitoring means comprises an optical fibre (82) extending from within the unit (10, 100).

16. The air conditioning system of claim 14, wherein said monitoring means comprises a light dependent resistor.

17. The air conditioning system of claim 14, 15 or 16, further comprising display means for displaying the operational status of the lamp.

18. The air conditioning system of claim 17, wherein the display means comprises a light emitting diode (80).

19. The air conditioning system of any preceding claim, wherein the housing (20, 200) is a scroll-shaped housing.

## Patentansprüche

1. Luftkonditionierungssystem aufweisend eine Gebläseeinheit (10, 100) zum Reinigen von Luft, wobei die Gebläseeinheit umfasst:
ein Gebläsegehäuse (20, 200);
einen rotierbaren Gebläsekörper (30, 300), der innerhalb des Gehäuses angeordnet ist, wobei der Gebläsekörper eine Strömungseinlassöffnung (36) aufweist; und
eine Luftreinigungslampe (40), die zumindest teilweise innerhalb der Strömungseinlassöffnung angeordnet ist, wobei, im Betrieb, durch die Strömungseinlassöffnung in das Gehäuse gesaugte Luft durch von der Lampe emittierte Strahlung gereinigt wird;
**dadurch gekennzeichnet, dass**:
der Gebläsekörper (30, 300) ein Bläserrad ist, das einen plissierten/gefalteten Filter umfasst, und ein photokatalytisches Material (60, 600) auf dem Gebläsekörper (30, 300) angeordnet ist.

2. Luftkonditionierungssystem nach Anspruch 1, wobei die innere Fläche des Gebläsegehäuses (20, 200) gerillt ist.

3. Luftkonditionierungssystem nach Anspruch 1 oder 2, wobei photokatalytisches Material (60, 600) auch auf zumindest einer inneren Fläche des Gebläsegehäuses (20, 200) angeordnet ist.

4. Luftkonditionierungssystem nach einem der vorangehenden Ansprüche, wobei die Lampe (40) eine ultraviolette Lampe umfasst.

5. Luftkonditionierungssystem nach Anspruch 4, wobei die ultraviolette Lampe (40) Licht emittiert, das eine Wellenlänge innerhalb des Bereichs von 100 nm bis 280 nm aufweist.

6. Luftkonditionierungssystem nach Anspruch 4, wobei die ultraviolette Lampe (40) Licht emittiert, das eine Wellenlänge von 253,7 nm aufweist.

7. Luftkonditionierungssystem nach Anspruch 1, wobei die Lampe (40) ultraviolettes Licht emittiert, das eine Wellenlänge innerhalb des Bereichs von 260 nm bis 400 nm aufweist.

8. Luftkonditionierungssystem nach Anspruch 1, wobei die Lampe (40) ultraviolettes Licht emittiert, das eine Wellenlänge innerhalb des Bereichs von 200 nm bis 260 nm, vorzugsweise 253,7 nm aufweist.

9. Luftkonditionierungssystem nach einem der vorangehenden Ansprüche, wobei die Lampe (40) länglich ist.

10. Luftkonditionierungssystem nach Anspruch 9, wobei die Lampe (40) im Wesentlichen entlang oder parallel zu der Rotationsachse des Gebläsekörpers (30, 300) ausgerichtet ist.

11. Luftkonditionierungssystem nach einem der Ansprüche 1 bis 8, wobei die Luftreinigungslampe (40) ringförmig ist.

12. Luftkonditionierungssystem nach einem der vorangehenden Ansprüche, wobei der Gebläsekörper (30, 300) ein radiales Gebläserad ist.

13. Luftkonditionierungssystem nach einem der vorangehenden Ansprüche, wobei das photokatalytische Material (60, 600) Titaniumdioxid umfasst.

14. Luftkonditionierungssystem nach einem der vorangehenden Ansprüche, des Weiteren umfassend Mittel zum entfernten Überwachen der Lampe (40), um deren Betriebsstatus zu bestimmen.

15. Luftkonditionierungssystem nach Anspruch 14, wobei das Überwachungsmittel eine optische Faser (82), die sich von innerhalb der Einheit (10, 100) erstreckt, umfasst.

16. Luftkonditionierungssystem nach Anspruch 14, wobei das Überwachungsmittel einen lichtabhängigen Widerstand umfasst.

17. Luftkonditionierungssystem nach Anspruch 14, 15 oder 16, des Weiteren umfassend Anzeigemittel zum Anzeigen des Betriebsstatus der Lampe.

18. Luftkonditionierungssystem nach Anspruch 17, wobei das Anzeigemittel eine Licht emittierende Diode (80) umfasst.

19. Luftkonditionierungssystem nach einem der vorangehenden Ansprüche, wobei das Gehäuse (20, 200) ein schneckenförmiges Gehäuse ist.

## Revendications

1. Système de climatisation comportant une unité de ventilateur (10, 100) permettant de purifier l'air, l'unité de ventilateur comprenant :
un logement de ventilateur (20, 200) ;
un corps de ventilateur rotatif (30, 300) disposé au sein du logement, le corps de ventilateur comportant une cavité d'admission d'écoulement (36) ; et
une lampe purificatrice d'air (40) disposée au moins partiellement au sein de la cavité d'admission d'écoulement, moyennant quoi, en utilisation, l'air tiré dans le logement à travers la cavité d'admission d'écoulement est purifié par un rayonnement émis par la lampe ;
**caractérisé en ce que** :
le corps de ventilateur (30, 300) est une roue de soufflante qui comprend un filtre plissé ; et
un matériau photocatalytique (60, 600) est disposé sur le corps de ventilateur (30, 300).

2. Système de climatisation selon la revendication 1, dans lequel la surface interne du logement de ventilateur (20, 200) est rainurée.

3. Système de climatisation selon la revendication 1 ou 2, dans lequel le matériau photocatalytique (60, 600) est également disposé sur au moins une surface interne du logement de ventilateur (20, 200).

4. Système de climatisation selon l'une quelconque des revendications précédentes, dans lequel la lampe (40) comprend une lampe à ultraviolet.

5. Système de climatisation selon la revendication 4, dans lequel la lampe à ultraviolet (40) émet de la lumière ayant une longueur d'onde dans la plage de 100 nm à 280 nm.

6. Système de climatisation selon la revendication 4, dans lequel la lampe à ultraviolet (40) émet de la lumière ayant une longueur d'onde de 253,7 nm.

7. Système de climatisation selon la revendication 1, dans lequel la lampe (40) émet de la lumière ultraviolette ayant une longueur d'onde dans la plage de 260 nm à 400 nm.

8. Système de climatisation selon la revendication 1, dans lequel la lampe (40) émet de la lumière ultraviolette ayant une longueur d'onde dans la plage de 200 nm à 260 nm, de préférence 253,7 nm.

9. Système de climatisation selon l'une quelconque des revendications précédentes, dans lequel la lampe (40) est allongée.

10. Système de climatisation selon la revendication 9, dans lequel la lampe (40) est alignée généralement le long de ou parallèle à l'axe de rotation du corps de ventilateur (30, 300).

11. Système de climatisation selon l'une quelconque des revendications 1 à 8, dans lequel la lampe purificatrice d'air (40) est annulaire.

12. Système de climatisation selon l'une quelconque des revendications précédentes, dans lequel le corps de ventilateur (30, 300) est une roue de ventilateur radiale.

13. Système de climatisation selon l'une quelconque des revendications précédentes, dans lequel le matériau photocatalytique (60, 600) comprend du dioxyde de titane.

14. Système de climatisation selon l'une quelconque des revendications précédentes, comprenant en outre un moyen pour surveiller à distance la lampe (40) afin de déterminer son état de fonctionnement.

15. Système de climatisation selon la revendication 14, dans lequel ledit moyen de surveillance comprend une fibre optique (82) s'étendant depuis l'intérieur de l'unité (10, 100).

16. Système de climatisation selon la revendication 14, dans lequel ledit moyen de surveillance comprend une résistance dépendant de la lumière.

17. Système de climatisation selon la revendication 14, 15 ou 16, comprenant en outre un moyen d'affichage permettant d'afficher l'état de fonctionnement de la lampe.

18. Système de climatisation selon la revendication 17, dans lequel le moyen d'affichage comprend une diode électroluminescente (80).

19. Système de climatisation selon l'une quelconque des revendications précédentes, dans lequel le logement (20, 200) est un logement en forme de spirale.
